(19) Europäisches Patentamt
European Patent Office
Office européen des brevets

(11) EP 0 799 262 B1

(12) **EUROPEAN PATENT SPECIFICATION**

(45) Date of publication and mention of the grant of the patent:
**03.01.2001 Bulletin 2001/01**

(21) Application number: **95943918.3**

(22) Date of filing: **19.12.1995**

(51) Int Cl.⁷: **C08G 59/02**, C08G 59/34, C07C 41/16, C07C 319/14, C07C 209/18

(86) International application number:
**PCT/US95/16632**

(87) International publication number:
**WO 96/20233 (04.07.1996 Gazette 1996/30)**

# (54) PROCESS FOR PREPARATION OF EPOXY COMPOUNDS

VERFAHREN ZUR HERSTELLUNG VON EPOXYVERBINDUNGEN

PROCEDE DE PREPARATION DE COMPOSES EPOXY

(84) Designated Contracting States:
**CH DE ES GB IT LI NL**

(30) Priority: **23.12.1994 US 363129**

(43) Date of publication of application:
**08.10.1997 Bulletin 1997/41**

(73) Proprietor: **THE DOW CHEMICAL COMPANY**
**Midland, Michigan 48674 (US)**

(72) Inventors:
- **AU, Andrew, T.**
  **Sugarland, TX 77479 (US)**
- **NAFZIGER, J., Lowell**
  **Lake Jackson, TX 77566 (US)**

(74) Representative: **Raynor, John**
**W.H. Beck, Greener & Co**
**7 Stone Buildings**
**Lincoln's Inn**
**London WC2A 3SZ (GB)**

(56) References cited:

| | |
|---|---|
| **EP-A- 0 368 421** | **EP-A- 0 370 946** |
| **US-A- 4 507 492** | **US-A- 4 740 330** |

- **JOURNAL OF ORGANOMETALLIC CHEMISTRY, vol. 326, 1987, pages c23-c28, XP002002526 MUZART J. ET AL: "Palladium complexes -KF/alumina catalysed exchange of allylic groups of esters with phenol"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention pertains to a process for the preparation of epoxy--containing compounds without the use of halogenated intermediates.

It is well-known to prepare epoxy compounds by reacting epihalohydrin with an active-hydrogen-containing compound and subsequently dehydrohalogenating the chlorohydrin intermediate product with a basic acting compound such as an alkali or alkaline earth metal hydroxide or carbonate. The five-step process from propylene to epoxy resin is usually as follows:

1) propylene + chlorine ---> allyl chloride
2) allyl chloride + water + chlorine ---> dichloropropanol
3) dichloropropanol + base ---> epichlorohydrin
4) epichlorohydrin + active-hydrogen-containing compound such as a bisphenol ---> chlorohydrin intermediate
5) chlorohydrin intermediate + basic acting compound ---> glycidyl ether epoxy resin

The process uses organohalide intermediates and makes a substantial quantity of organohalide by-products, which require special handling.

EPO Publication 0 368 421 A2 (16-May-90) has proposed the following alternative synthesis to make epoxy resins:

(1) reacting methallyl halide with a polyhydric phenol to make the poly(allyl ether) of the polyhydric phenol; and
(2) oxidizing the allyl groups in the poly(allyl ether) to make the poly(glycidyl ether) of the polyhydric phenol.

This process suffers from poor yields and still requires halogen in the process. It is impractical as a route to intermediates and to make commercial quantities of epoxy resin.

Japanese Patent Application 60-124615 to Sumitomo Bakelite teaches the following alternative synthesis to make epoxy resins:

(1) reacting allyl halide with a polyhydric phenol to make the poly(allyl ether) of the polyhydric phenol; and
(2) oxidizing the allyl groups in the poly(allyl ether) to make the poly(glycidyl ether) of the polyhydric phenol.

The products of this process contain high levels of side-products, and the process still requires halogen. It is impractical as a route to intermediates and to make commercial quantities of epoxy resin.

What is needed is a practical process to make epoxy resins without resort to halogenated intermediates.

The present invention is a process to make an epoxide compound containing the steps of:

(1) providing an allyl reagent;
(2) allylating one or more compounds containing at least one active-hydrogen atom using the allyl reagent from step (1), whereby allyl ether, allyl thioether or allyl aryl amine compound is formed; and
(3) converting the allyl groups on the compound from step (2) to epoxide groups, whereby a glycidyl ether, glycidyl thioether or glycidyl aryl amine compound is formed,

characterized in that the allyl reagent is an allyl carboxylate and in that step (2) is catalyzed by a transition metal catalyst.

Using preferred embodiments of the present invention, the ultimate production of the epoxy-containing compounds can be accomplished efficiently in three steps starting with the basic raw material, propylene without the use of halogenated organic intermediates (aside from halogens which may be present on the active-hydrogen-containing compound). A preferred three-step process is summarized as follows:

1) Propylene + oxygen + carboxylic acid + catalyst---> allyl carboxylate;
2) Allyl carboxylate + active-hydrogen-containing compound such as a bisphenol ---> allyl derivative of active-hydrogen-containing compound; and
3) Allyl derivative of active-hydrogen-containing compound + peroxy oxidant---> glycidyl derivative of active-hydrogen-containing compound.

As a further advantage, the compounds prepared by the process of the present invention may have none or very little total organic halides present except for halogen introduced by the active-hydrogen-containing compound. For instance, the process of the present invention is suitable for the preparation of the diglycidyl ether of tetrabromobisphenol A; however, it will not be essentially free of organic halides because of the bromine present in the tetrabromobisphenol A starting material. Epoxy resins which contain none or very little total organic halides are particularly suitable for electronic applications, metal primer coatings, cathodic electrodeposition coatings and powder coatings.

The term "catalyst efficiency" or "turnover rate" is defined by the following equation:

$$\text{Catalyst Efficiency or Turnover Rate} = \frac{\text{(Equivalents of Active Hydrogen Converted Formed)}}{\text{(Mole of Catalyst)/Hour}}$$

Step (1) Providing an Allyl Reagent

The allyl reagent used in the present invention is an allyl carboxylate and most preferably it is allyl acetate.

The allyl reagent is preferably employed in amounts such that the equivalent ratio of allyl reagent to active-hydrogen atom contained in the active-hydrogen-containing compound is from 0.1:1 to 500:1, more preferably from 0.5:1 to 50:1, and most preferably from 1:1 to 5:1. At ratios significantly above 1:1, the allyl reagent acts as both a reagent and a solvent.

At ratios below 0.1:1, the conversion to the desired product is very low and the excess active-hydrogen-containing reactant must be recovered and recycled in the process.

Allyl Carboxylates

The allyl carboxylates which are useful as allyl reagents are preferably represented by the following Formula I:

Formula I

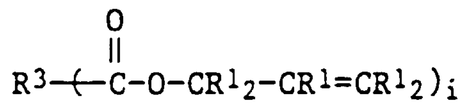

wherein $R^1$ is hydrogen or an alkyl group having from 1 to 12 carbon atoms which may optionally be linked to another $R^1$ group to form a ring structure, and
$R^3$ is hydrogen or an alkyl, cycloalkyl or aryl group having from 1 to 10 carbon atoms and "i" equals on average 1 to 3.

Preferably, the allyl carboxylate is allyl formate, allyl acetate, allyl propionate, allyl benzoate, or a diallyl carboxylate such as oxalate, glutarate, succinate, or any combination thereof. Most preferred as the allyl carboxylate is allyl acetate.

Preparation of Allyl Carboxylates

Certain useful allyl carboxylates, such as allyl acetate, are commercially available. Allyl carboxylates can also be prepared by the oxidation of propylene or corresponding olefins in the presence of oxygen, a carboxylic acid (such as formic acid, acetic acid or propionic acid) and a palladium catalyst as described in U.S. Patent 3,970,713. The reaction is conducted at temperatures of from 50°C to 250°C. In the reaction, from 5 to 20 moles of water per mole of acid, from 1 to 5 moles of oxygen per mole of acid and from 4 to 40 moles of olefin per mole of oxygen are employed This reaction proceeds readily in one step from the olefin to the allyl carboxylate without the use or production of halogenated materials.

Step (2) Preparation of the Allyl Derivative of Active-Hydrogen-Containing Compounds

The allyl derivative of the active-hydrogen-containing compound is prepared by reacting an active-hydrogen-containing compound with an allyl carboxylate as the allylating agent in the presence of a transition metal catalyst, optionally but preferably, in the presence of at least one complexing agent for the catalyst and in the presence of at least one base compound and further, optionally but preferably, in the presence of one or more suitable solvents.

The reaction is preferably conducted at a temperature of from 0°C to 200°C, more preferably from 25°C to 150°C, more preferably from 50°C to 110°C, and preferably at a pressure sufficient to keep the reaction mixture in liquid form. The reaction time to completion is preferably from 0.1 to 72 hours, more preferably from 0.1 to 48 hours, and most preferably from 0.1 to 24 hours, Optimum temperature and times for particular reactants will vary depending upon the reactivity of the particular reactants, solvents and catalysts employed; however, such optimum temperatures and times can readily be determined in the laboratory with minimum effort.

At temperatures below about 0°C, the reaction proceeds at a slower rate and costs for cooling are incurred.

At temperatures above about 200°C, costs for providing the increased temperature are incurred.

Particular pressures are not deemed to be determinative of any appreciative effects on the process; however, pressures are preferably employed which will keep the reaction in the liquid phase.

The allyl derivative of the active-hydrogen-containing compound can also be prepared by reacting an active-hydrogen-containing compound with an allyl carbonate as the allyl reagent in the presence of a catalyst such as, for example, ruthenium, rhodium, rhenium, palladium, molybdenum, tungsten, nickel, platinum, or any combination thereof; and optionally in the presence of one or more suitable solvents. Reaction temperatures are preferably from -20°C to 250°C, more preferably from 20°C to 200°C, and most preferably from 30°C to 120°C. The reaction time will vary with the temperature and the particular catalyst and amount of catalyst employed; however, suitable reaction times include from 0.1 to 100, preferably from 0.1 to 50, and more preferably from 0.1 to 10 hours. This process employing phenols, thiophenols and aryl amines as the active-hydrogen-containing compound is described by Edmund P. Woo in U.S. Patent 4,507,492.

Suitable allyl derivatives which can be prepared in the process of the present invention include, but are not limited to, for example, those represented by any one of the following Formulas III to X:

Formula III $R^2\text{-}(Z)_a$

Formula IV

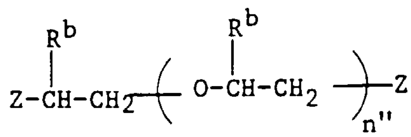

Formula V

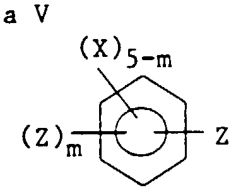

Formula VI

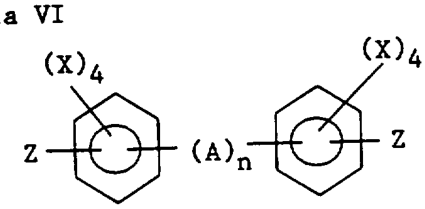

Formula VII

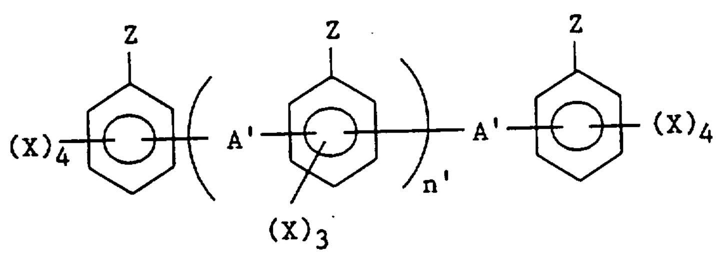

Formula VIII

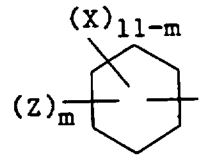

Formula IX

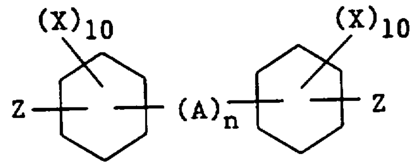

Formula X

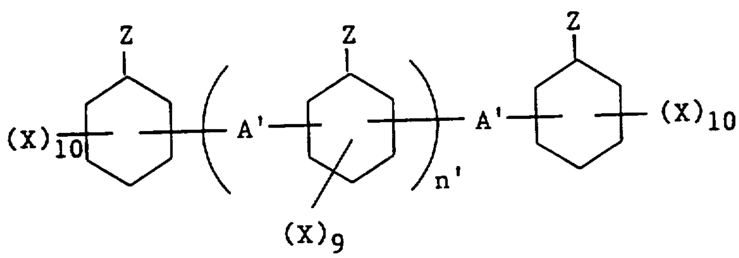

wherein each A is preferably independently a divalent hydrocarbyl group having from 1 to 20, preferably from 1 to about 10, and more preferably from 1 to 3 carbon atoms, -O-, -S-, -S-S-, -SO- or $-SO_2-$, -CO-, -COO-, -C=CR-, -C-O-C-, $CHX^a$ ($X^a$ is an inert moiety which is not hydrogen or hydrocarbyl, such as halogen or cyanide); A' is a divalent hydrocarbyl group having from 1 to 10, preferably from 1 to 4, more preferably from 1 to 2 carbon atoms; each X is independently a hydrogen, a monovalent hydrocarbyl or hydrocarbyloxy group having from 1 to 20, preferably from 1 to 10, and more preferably from 1 to 4 carbon atoms, a halogen atom (preferably chlorine or bromine), or a $-NO_2$ group; each Z is preferably independently a

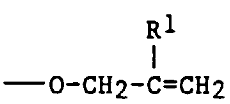 , 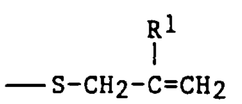 , 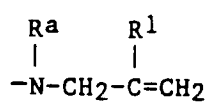

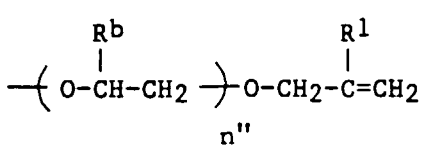 , 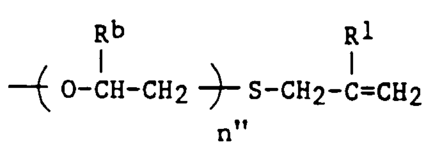 ,

group; $R^a$ is hydrogen, an alkyl group having from 1 to 4 carbon atoms or a $-CR^1_2-C(R^1)=CR^1_2$ group; $R^b$ is hydrogen, an alkyl group or a hydrocarbyloxy group having from 1 to 6 carbon atoms; $R^1$ is preferably hydrogen or an alkyl group having from 1 to 4 carbon atoms; $R^2$ is a monovalent or polyvalent aromatic, aliphatic or cycloaliphatic hydrocarbon group having from 2 to 20, preferably from 3 to 10, and more preferably from 4 to 8 carbon atoms; "m" has a value of

zero or 1; "n" has a value of zero or 1; "n'" preferably has a value from 0.01 to 10, more preferably from 0.5 to 6, and most preferably from 1 to 4; 'n''' has preferably a value from zero to 50, more preferably from zero to 20, and most preferably from zero to 6; and "a" is preferably 1 to 10, more preferably 2 to 6, and most preferably 2 to 4.

Active-Hydrogen-Containing Compounds

The active-hydrogen-containing compound preferably contains at least one hydroxyl group, thiol group, or primary or secondary aryl amine group per molecule. It more preferably contains on average more than one such group, and most preferably contains on average at least about 1.8 such groups. It preferably contains no more than about 10 such groups. It more preferably contains hydroxyl groups. Examples of suitable active-hydrogen--containing compounds include mono-, di- or multi-functional aliphatic, cycloaliphatic or aromatic hydroxyl-containing; thiol-containing; primary and secondary aromatic amine--containing compounds or any combination of such compounds. Particularly suitable examples of active-hydrogen-containing compounds include those represented by any one of Formulas III or V to XI and the following Formula XII:

Formula XII

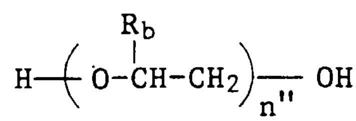

or any combination of any two or more such active-hydrogen-containing compounds wherein A, A', $R^a$, $R^b$, $R^1$, $R^2$, X, m, n, n' and n" are as defined above and Z is -OH, -SH; $-NH_2$, or $-NH(R^4)$, wherein $R^4$ is hydrogen or a saturated or unsaturated aliphatic group having from 1 to 20, preferably from 1 to 10, and more preferably from 1 to 6 carbon atoms.

Hydroxyl-Containing Compounds Which Are Useful as Active-Hydrogen-Containing Compounds

The hydroxyl-containing compounds can be aliphatic, cycloaliphatic or aromatic compounds.

Particularly suitable aromatic hydroxyl-containing compounds include, for example, phenol, cresol, catechol, resorcinol, bisphenol A (p,p'-isopropylidene diphenol), bisphenol F (p,p'-methylene diphenol), bisphenol K (p,p'-diphenolcarbonyl), dihydroxy-α--methylstilbene, dihydroxybiphenyl, bis(hydroxyphenyl)fluorene, phenol-aldehyde novolac resins, alkyl substituted phenol-aldehyde novolac resins, dihydroxynaphthalenes or any combination thereof. Preferred aromatic hydroxyl-containing compounds include, for example, bisphenol A, bisphenol F, dihydroxy-a-methylstilbene, phenol-formaldehyde novolac resins, cresol-formaldehyde novolac resins or any combination of any two or more of such compounds thereof.

The active-hydrogen-containing compound is preferably a bisphenol, and is most preferably bisphenol A.

Suitable aliphatic hydroxyl-containing compounds include, for example, propanol, butanol, hexanol, octanol, butanediol, hexanediol, octanediol, polyoxyalkylene glycols which preferably have a weight average molecular weight of from 106 to 50,000, preferably from 106 to 5,000, and more preferably from 106 to 2500, or any combination thereof.

Particularly suitable cycloaliphatic hydroxyl-containing compounds include, for example, cyclopentanol, cyclohexanol, methylcyclohexanol, dihydroxycyclohexane, norbornanediol, cyclohexanedimethylol, dicyclopentadienediol, vinylcyclohexene derived diols, or any combination thereof.

Thiol Compounds Which Are Useful as Active-Hydrogen-Containing Compounds

The thiol-containing compounds can be aliphatic, cycloaliphatic or aromatic compounds. Particularly suitable thiol compounds include, for example, thiophenol, bisthiophenol, thiobisphenol A, thiophenol-formaldehyde novolac resins, thiocresol-formaldehyde novolac resins, thionaphthol, or any combination of any two or more compounds thereof.

Primary and/or Secondary Aromatic Amine-Containing Compounds Which Are Useful as Active--Hydrogen-Containing Compounds

Particularly suitable primary or secondary aromatic amine-containing compounds include, for example, diaminobenzene, aniline, N-methyl aniline, methylene dianiline, hydroxyaniline, bis-aniline, diaminotoluene, amino-naphthylene or any combination thereof.

Base Compounds

Suitable base compounds which are used in step (2) of the present invention with allyl carboxylates include, for example, ammonium, alkali and alkaline earth metal bicarbonates, carbonates, phosphates, citrates, acetates, hydroxides, hydrides or alkoxides, such as sodium carbonate, potassium carbonate, lithium carbonate, calcium carbonate, sodium hydroxide, potassium hydroxide, lithium hydroxide, calcium hydroxide, sodium bicarbonate, potassium bicarbonate, lithium bicarbonate, calcium bicarbonate, or any combination of any two or more such base compounds. Particularly suitable base compounds include, for example, potassium carbonate, sodium carbonate, potassium hydroxide, sodium hydroxide, sodium bicarbonate or any combination of any two or more such base compounds. Preferred base compounds include sodium carbonate, sodium bicarbonate and sodium hydroxide.

Suitable resinous bases include, for example, the hydroxide or carbonate quaternary ammonium or amino form of ion exchange resins, particularly styrene/divinyl benzene-based ion exchange resins.

The base compounds are preferably employed in amounts of from 0.8 to 10, more preferably from 1 to 2.0, and most preferably from 1 to 1.2 equivalents of base per active hydrogen of the material to be allylated.

Buffering Agents

Strong base compounds such as hydroxides or alkoxides, which are used with allyl carboxylates, are preferably modified with buffering agents such as, for example, alkali or alkaline earth metal bicarbonates, alkali or alkaline earth metal carbonates, carbon dioxide, alkali or alkaline earth metal hydrogen phosphates, or alkali or alkaline earth metal borates. For example, sodium carbonate may be formed *in situ* using sodium hydroxide and carbon dioxide. The buffering agents are preferably employed in amounts such that the pH of the aqueous portion, if any, in the reaction mixture is from 7 to 13, more preferably from 8 to 12, and most preferably from 9 to 11. As an alternative method to control pH, unbuffered bases may be added continuously or in stages over a period of time.

Catalysts

The reaction of step (2) is run in the presence of a transition metal catalyst. Suitable catalysts contain, for example, rhodium, ruthenium, rhenium, palladium, iridium, tungsten, molybdenum, chromium, platinum, nickel, copper, osmium, iron, either in an unoxidized state as the free metal or a complex or in an oxidized state as their salts such as, for example, carboxylates, halides, oxides, nitrates or sulfates. The preferred catalysts contain palladium, platinum, nickel or molybdenum. The most preferred catalysts contain palladium. Salts are preferably acetate, chloride, nitrate or sulfate salts.

When the catalyst is employed in its free metal state it is preferably supported on a support material such as, for example, carbon, charcoal, activated charcoal, silica, alumina, zeolite, organosol gel, clay or any combination of any two or more of such support materials.

When the catalyst is in the form of a complex or a salt, it is preferably not supported.

Homogeneous catalysts are preferably employed in amounts of from 0.005 to 200, more preferably from 0.01 to 50, more highly preferably from 0.05 to 10, and most preferably from 0.1 to 2 mmoles per equivalent of active-hydrogen atom contained in the active-hydrogen-containing compound. The quantity of homogeneous catalyst is preferably about the minimum required to provide a reaction at the desired speed.

The supported catalyst preferably contains from 0.1 to 80, more preferably from 0.5 to 50, and most preferably from 0.5 to 10 percent by weight catalyst. The supported catalyst preferably contains at least about 0.005 mmoles of transition metal per equivalent of active hydrogen, more preferably at least about 0.01 mmoles, more highly preferably at least about 0.05 mmoles, and most preferably at least about 0.1 mmoles. The maximum concentration is not critical. When the catalyst is employed as a supported, heterogeneous catalyst, the reaction can be conducted in a fixed bed or suspended in the liquid reaction mixture.

Complexing Agents

Complexing agents are preferably employed to serve as ligands to stabilize and to enhance the activity of the metal catalyst. The catalyst can be complexed with the complexing agent prior to addition to the reaction mixture or the catalyst complex can be formed *in situ* by adding the catalyst and the complexing agent separately to the reaction mixture.

Suitable complexing agents include, for example, organic mono- or diphosphines, organic phosphites, organic stibines, oximes, organic arsines, diamines, and dicarbonyl compounds or any combination of any two. Particularly

suitable complexing agents include, for example, triphenyl phosphine, tris-tolyl phosphine, diphenylmethyl phosphine, diphenylphosphino ethane, or any combination of any two or more such complexing agents. The preferred complexing agents include, tris-tolyl phosphines, triphenyl phosphine and diphenylphosphino ethane with triphenyl phosphine being most preferred. The complexing ligand may also be supported on a resinous backbone.

Aqueous soluble complexing agents, such as sulfonated triphenylphosphines and their salts, may also be used. This type of ligand will have the advantage of being water soluble and easily washed out/separated from the organic product layer.

The complexing agents are preferably employed in amounts of from about 10 to 1000, more preferably from 50 to 500, and most preferably from 200 to 400 mole per 100 moles of the catalyst excluding any support material.

## Solvents

Suitable solvents which can optionally, but preferably, be employed in step (2) of the present invention include, for example, water, excess amounts of allyl carboxylate, and oxygenated hydrocarbons (such as alcohols, ethers, glycols, glycol ethers, esters and ketones). Other solvents including nitroalkanes, cyanoalkanes, alkyl sulfoxides, amides, aromatic hydrocarbons, halogenated hydrocarbons or any combination of any two or more such solvents can also be used. Particularly suitable solvents include, for example, water, allyl acetate, acetonitrile, acetone, methyl ethyl ketone, ethyl acetate, dimethylformamide, dimethylsulfoxide, nitromethane, tetrahydrofuran, ethanol, isopropanol, t-butanol, t-amyl alcohol, glymes or any combination thereof. The preferred solvents are water, allyl acetate, isopropanol, t-butanol, t-amyl alcohol, acetone, acetonitrile, glycols and glycol ethers or any combination of any two or more such solvents. A more highly preferred system is a solvent system containing an aqueous phase and an organic phase. The organic phase preferably uses excess allyl carboxylate compound as a solvent. The mixture is preferably mixed vigorously to bring the two phases into intimate contact with each other. The weight ratio of aqueous to organic phase is preferably 9:1 to 1:9.

The solvents are preferably employed in amounts of from zero to 100, more preferably from 0 to 20, and most preferably from 0 to 10 parts by weight based upon the weight of active-hydrogen-containing component.

## Step (3) Epoxidation

The allyl ether, allyl thioether and allyl aryl amine compounds made in step (2) are converted to epoxy compounds by the methods known in the art for epoxidizing ethylenic or olefinic double bonds with peroxygen compounds. Suitable methods include those disclosed in Epoxy Resins Chemistry and Technology, 2nd Ed., 1988, Clayton A. May, Marcel Dekker, Inc., Chapter 2, part III, pp. 15-46; Buchler et al., U.S. Patent 4,973,718 (November 27, 1990); Schmidt et al., U.S. Patent 4,845,252 (July 4, 1989); and Kim, U.S. Patent 4,418,203 (November 29, 1983).

One method to perform such epoxidations is conducted by reacting the allyl ethers with a peroxygen compound such as peracetic acid, t-butyl hydroperoxide, or hydrogen peroxide in the presence of suitable catalysts.

For example, hydrogen peroxide epoxidation is preferably conducted in the presence of $Na_2WO_4/H_3P0_4/$ quaternary ammonium salt or trioctyl methyl ammonium nitrate at temperatures of from 0°C to 100°C, more preferably from 20°C to 60°C, and most preferably from 35°C to 50°C.

The peracetic acid epoxidation is preferably conducted at temperatures of from 20°C to 200°C, more preferably from 40°C to 100°C, and most preferably from 40°C to 80°C.

A highly preferred summary of the entire three-step process from propylene to epoxy resin is as follows:

1) propylene + oxygen + carboxylic acid + catalyst---> allyl carboxylate;
2) allyl carboxylate + active-hydrogen-containing compound such as bisphenol A ---> allyl derivative of active-hydrogen-containing compound; and
3) allyl derivative of active-hydrogen-containing compound + peroxy oxidant ---> glycidyl derivative of active-hydrogen-containing compound.

The carboxylic acid is most preferably acetic acid, and the allyl carboxylate is most preferably allyl acetate. The active-hydrogen-containing compound is most preferably a bisphenol, the allyl derivative is most preferably a diallyl derivative, and the glycidyl ether derivative is most preferably a diglycidyl ether derivative.

The epoxy resin made by the present invention preferably contains on average at least about one glycidyl ether, glycidyl thioether, or glycidyl aryl amine group per molecule; more preferably contains on average more than one such group per molecule, and most preferably contains on average at least about 1.5 such groups per molecule. It preferably contains on average no more than about 10 such groups per molecule, more preferably no more than about 3, and most preferably no more than about 2.1. The functional groups are more preferably glycidyl ether groups. The resin preferably contains less than 600 ppm of organic halogen, more preferably less than about 300 ppm, more

highly preferably less than about 150 ppm, and most preferably less than about 50 ppm total organic halogen. These amounts of "total organic halogen" exclude any halogen atoms which were introduced as part of the active-hydrogen-containing compound.

Theoretically, the process of the present invention, which does not include the use of an epihalohydrin, may result in products totally free of any organic halogen, but some organic halogen may be found in the product due to contamination of raw materials employed including, but not limited to solvents, active-hydrogen-containing compounds, catalysts, and bases. The epoxy resins prepared by the process of the present invention are useful in such applications as, for example, electrodeposition and powder coatings, encapsulants, molding, casting, composites and laminates, and adhesives. The epoxy resins prepared by the process of the present invention based on aromatic hydroxyl-containing compounds, particularly those based on cresol-formaldehyde novolac resins are particularly useful in the preparation of electrical laminates and encapsulated electronic components, cathodic electrodeposition and powder coatings since halogen atoms are known to be undesirable-components in epoxy resins used in these applications.

The following examples are illustrative of the invention and should not be interpreted as limiting it in any way. Unless stated otherwise all parts and percentages are given by weight.

In the following examples, the amount of total halide is determined by digesting the sample with caustic (NaOH) and titrating with silver nitrate.

In the following examples, the epoxide equivalent weight (EEW) is determined by titration with an acid.

Example 1

A. Preparation of Bisphenol A Diallyl Ether From Bisphenol A and Allyl Acetate Using a Heterogeneous Palladium Catalyst

To a mixture of 11.4 g (0.05 mole) of bisphenol A, 0.1 g of 5 percent palladium (0.0469 mmole) on charcoal, 0.1 g (0.38 mmole) of triphenyl phosphine, and 14.5 g (0.105 mole) of potassium carbonate in 30 mL of isopropanol under a nitrogen atmosphere was added 11.0g (1.1 equivalent.) of allyl acetate and the mixture stirred at 85°C. After 4 hours, both thin layer chromatography and high performance liquid chromatography analysis showed that the reaction was practically completed and showed only one product peak. The catalyst turnover rate is calculated to be 533 equivalent. of active hydrogen/mole of catalyst/hour.

To the mixture prepared above, 30 mL of water and 100 mL of ethyl acetate were added. The mixture was shaken well. The mixture was filtered and the organic layer was separated, dried and concentrated in vacuo at 2 mm Hg and 50°C to yield 15.3 g of a colorless oil representing a yield of 99.4 percent based on bisphenol A. Both infrared (IR; with no OH absorption) and nuclear magnetic resonance (NMR; appearance of an appropriate quantity of corresponding allylic protons) spectra were consistent with the desired product of bisphenol A diallyl ether and identical to a known sample of diallyl ether of bisphenol A which was derived from bisphenol A and allyl chloride.

B. Epoxidation of Bisphenol A Diallyl Ether

To a solution of 12.5 g of diallyl ether of bisphenol A (40.6 mmole) and 3.2 g (7.6 mmole) of trioctylmethylammonium nitrate in 30 mL of toluene were added an aqueous solution of 2.6 g of sodium tungstate (8.8 mmole), 1.6 g of phosphoric acid (16.3 mmole) and 16 g (about 0.15 mole) of 30 percent hydrogen peroxide in 32 mL of water (final pH of 2.05). The mixture was stirred at 40°C and high performance liquid chromatography was used to monitor the progress of the reaction. After 4 days, the reaction was stopped when total disappearance of the allyl ether and the formation of a new product peak were observed. The aqueous layer was separated and extracted twice with 20 mL portions of toluene. The organic reaction mixture was combined with the extracts, washed twice with 30 mL portions of water, and dried over magnesium sulfate and then concentrated in vacuo resulting in 17.0 g of a brown oil.

This crude brown oil was passed through a column of 250 g of silica gel packed in 20 percent of acetone in hexane. Elution of this column with a gradient of 20 to 50 percent acetone in hexane yielded 9.5 g of a light yellow oil. Spectral data (IR and NMR; NMR: absence of olefinic protons and appearance of epoxide protons) were consistent with the desired product (the diglycidyl ether of bisphenol A). Furthermore, both thin layer chromatography and high performance liquid chromatography showed identical retention times with known samples of diglycidyl ether of bisphenol A. Analysis showed that the product obtained contained 26 ppm of total chloride and had an epoxy equivalent weight of 182.

Example 2

A. Preparation of Bisphenol A Diallyl Ether From Bisphenol A and Allyl Acetate Using Homogeneous Palladium Catalyst

To a glass reactor equipped with glass baffles under a nitrogen atmosphere was added a solution of 35.0 g (0.875 mol) of sodium hydroxide in 300 mL of water. Then 40 g of dry ice was gradually added until a pH of 10.5 was obtained. Then 45.6 g (0.2 mole) of bisphenol A was added. After stirring for 20 minutes, the mixture was heated to 85°C and 80.0 g (0.8 mole) of allyl acetate, 0.028 g (0.125 mmole) of palladium acetate and 0.162 g (0.617 mmole) of triphenyl phosphine were added. After stirring rapidly (about 600 rpm) for 15 minutes, the reaction was >99 percent complete and the high performance liquid chromatogram showed cleanly one product peak. The stirring was stopped and the organic layer was separated and concentrated in vacuo to yield 58.61 g of desired product. The yield was 95.2 percent. The catalyst turnover rate was calculated to be 12,800/hour.

Repeating the above example using a stirring rate of about 60 rpm instead of about 600 rpm the conversion was only 14.4 percent after 15 minutes.

B. Epoxidation of Diallyl Ether of Bisphenol A

A 50 mL flask was equipped with a thermometer, condenser, a magnetic stirrer and a pH meter. A mixture containing: 22.5 g (0.55 mole) of acetonitrile, 109 of methanol, 0.15 g (1.06 mmole) of dibasic sodium phosphate, 1.69 g (5.5 mmole) of diallyl ether of bisphenol A from Step (A), and 1.12 g of 30 percent aqueous hydrogen peroxide solution (containing 9.9 mmole of hydrogen peroxide) was added to the flask. The temperature was raised to 50°C with stirring. After 6 hours, 1.12 g of 30 percent hydrogen peroxide solution was added, and the temperature was raised to 55°C. After another 6 hours (12 total), 1.68 g of 30 percent hydrogen peroxide solution was added, and the temperature was raised to 60°C. After another 7 hours (19 total), the reaction was terminated. The conversion of diallyl ether was about 100 percent. High-performance liquid chromatography indicates that the ratio of diglycidyl ether of bisphenol A to (monoglycidyl ether-monoallyl ether of bisphenol A + diallyl ether of bisphenol A) is 89.5:10.5.

Example 3

A. Preparation of Allyl Phenolic Novolac

To a mixture of 6.2 g (20 mmole) of cresol novolac (average M.W. = 310, hydroxy equivalent weight = about 124, average functionality = 2.5), 8.0 g (57.9 mmole) of potassium carbonate, 50 mg of 5 percent palladium (0.024 mmole) on charcoal, and 20 mg (0.08 mmole) of triphenyl phosphine in 25 mL of isopropanol, was slowly added 6.0 g (60 mmole) of allyl acetate. After the resultant mixture was stirred at 85°C for 8 hours, high performance liquid chromatography showed that the reaction was complete. Water (30 mL) and toluene (30 mL) were added to the mixture. After extracting, the toluene layer was separated and the aqueous layer was again extracted with another 30 mL of toluene. After separation, the toluene extracts were combined, dried over magnesium sulfate and concentrated in vacuo to a viscous pale yellow oil (7.8 g, about 95 percent yield). Spectral data of this material were consistent with the desired allylated product. The NMR spectrum showed the presence of the vinylic hydrogens and the allylic hydrogens while the IR spectrum showed the absence of OH absorption.

B. Epoxidation of Allyl Phenolic Novolac

An aqueous solution containing 2.65 g (8 mmole) of sodium tungstate dihydrate, 1.6 g (16 mmole) of phosphoric acid and 8.0 g (0.165 mole) of 70 percent hydrogen peroxide in 28 mL of water (mixture pH = 2.05) was added to a 24 mL toluene solution containing 12.6 g (0.1 equiv.) of allylated cresol novolac prepared in (A) above and 1.6 g (4.5 mmole) of trioctylmethylammonium nitrate. The mixture was stirred at 45°C for 28 hours and was stopped when almost all starting material had disappeared. The mixture was cooled and the organic layer separated and washed 3 times with 50 mL of water. It was then dried over magnesium sulfate, filtered and concentrated in vacuo to give 13.6 g of a viscous amber liquid.

To remove the ammonium nitrate from the crude product, the material was extracted with 5 mL of methanol by warming up with stirring, cooling to room temperature and decanting the methanol phase. The process was repeated four times. The product was then concentrated with roto-evaporator (< 100°C/2 mm Hg), yielding 9.1 g of desired cresol epoxy novolac {analysis: total organic Cl = 1 ppm, percent epoxide = 19.12 percent, EEW = 224.9).

Example 4

A. Allylation of Benzyl Alcohol

A mixture of benzyl alcohol (10.8 g, 0.1 mole), allyl acetate (10.5 g, 0.105 mole) and potassium carbonate (15 g, 0.109 mole) in 15 mL of water was stirred under nitrogen for 15 minutes. Then 10 mg (0.045 mmole) of palladium acetate and 30 mg (0.115 mmole) of triphenyl phosphine were added and the mixture was stirred at 85°C. After 48 hours, the reaction was 65 percent completed and the reaction rate became quite sluggish. After cooling, 50 mL of water and 50 mL of toluene were added for extraction. The organic extract was separated and washed twice with 30 mL of water, dried over magnesium sulfate and concentrated in vacuo yielding 14.2 g. This light brown liquid was distilled and collected at 71°C to 74°C/13 mm Hg, yielding 8.5 g of a colorless liquid (NMR and IR spectra were consistent with the desired benzyl allyl ether).

B. Epoxidation of Benzyl Allyl Ether

An aqueous solution containing 1.32 g (0.004 mole) of sodium tungstate dihydrate, 1.0 g (0.012 mole) of phosphoric acid and 12.5 g (0.11 mole) of 30 percent hydrogen peroxide in 18 mL of water (mixture pH = 1.98) was added to a 24 mL toluene solution containing 8.5 g (0.574 mole) of benzyl allyl ether and 1.0 g (2.3 mmole) trioctylmethylammonium nitrate. The mixture was stirred at 45°C for 36 hours and was stopped when almost all starting material had disappeared. The mixture was cooled and the organic layer was separated and washed three times with 50 mL of water. It was then dried over magnesium sulfate, filtered and concentrated in vacuo to give 12.2 g of a viscous amber liquid.

To remove the ammonium nitrate from the crude product, the material was chromatographed over 200 g of silica gel packed in 10 percent acetone in hexane. Elution of this column with a gradient of 10 to 20 percent acetone/hexane yielded 8.2 g of material analyzed to have total organic chloride of 199 ppm and an EEW of 164.

Example 5

A. Allylation of t-Butylphenol

A mixture containing 2500 g of deionized water and 352.1 g (8.8 mole) of sodium hydroxide was added to a 5 L glass reactor having a stirrer, a condenser, a temperature controller, heat lamps, and a nitrogen pad. Dry ice was added until the pH of the solution was 10.8, and then 300 g (2.0 mole) of t-butylphenol was added. The mixture was heated to 83°C with stirring, and 400 g (4.0 mole) of allyl acetate was added. A mixture containing 1.625 g (6.2 mmole) of triphenylphosphine and 0.280 g (1.25 mmole) of palladium acetate was added immediately. The mixture foamed for a short time. After the foaming had decreased, an additional 300 g (2.0 mole) of t-butylphenol and 400 g (4.0 mole) of allyl acetate were added. The mixture was stirred for 30 minutes, and then cooled to 50°C. The organic layer was separated and washed with two 1000 mL portions of 1 percent aqueous EDTA solution and two 500 mL portions of water. Anhydrous sodium sulfate was added to the supernatant until clear, and then 5 g of activated carbon was added. The mixture was vacuum filtered The filtrate was stripped and concentrated at 1 to 2 mm of vacuum and 60°C to give 808 g of crude t-butylphenyl allyl ether The crude product was further distilled at 85°C to 90°C and 3 to 3.5 mmHg pressure for further purification.

B. Epoxidation of t-Butylphenyl Allyl Ether

A 50 mL flask was equipped with a thermometer, condenser, a magnetic stirrer and a pH meter. A mixture containing 22.5 g (0.55 mole) of acetonitrile, 10 g of methanol, 0.135 g (0.95 mmole) of dibasic sodium phosphate, and 2.8 g of 30 percent hydrogen peroxide solution (containing 25 mmole of hydrogen peroxide) was added to the flask, and the temperature was raised to 45°C with stirring. A 1.76 g (9.25 mmole) quantity of t-butylphenyl allyl ether was added. The reaction was continued for 24 hours at 45°C, and 2.8 g portions of 30 percent hydrogen peroxide solution (containing 25 mmole of hydrogen peroxide) were added at 6 and 20 hours. The pH of the solution was maintained between 8.0 and 9.5 by adding a few drops of 0.5 N sodium hydroxide solution as needed. Gas chromatograph analysis showed that the ratio of t-butylphenyl glycidyl ether to t-butylphenyl allyl ether was 97:3.

Referential Examples 6-11 - Allylation of Phenol

Several different mixtures of phenol and allyl acetate were reacted together under inert atmosphere in 3-neck, round-bottom flasks fitted with a gas inlet, reflux condenser and a thermometer equipped with a temperature controller.

The reactor contents were heated using an infrared lamp to 85°C and stirred using a magnetic stirrer. The reagents, the reaction time, and the ratio of allyl phenyl ether to phenol in the product mixture are shown in Table I. Analyses were obtained using gas chromatography.

TABLE I

| Example | 6 | 7 | 8 | 9 | 10 | 11 | A* |
|---|---|---|---|---|---|---|---|
| Phenol (moles) | 0.05 | 0.02 | 0.1 | 0.1 | 0.05 | 0.02 | 0.02 |
| Allyl Ace. (moles) | 0.06 | 0.022 | 0.12 | 0.12 | 0.06 | 0.022 | 0.022 |
| Solvent/mL | IPA/15 | IPA/8 Water/2 | Water/16 | Water/16 | Water/10 | IPA/100 | IPA/100 |
| Base | $K_2CO_3$ | $K_2CO_3$ | $NaOH/CO_2$ | $NaOH/CO_2$ | $K_2CO_3$ | $K_2CO_3$ | KOH |
| Moles of Base | 0.054 | 0.023 | 0.11 | 0.11 | 0.054 | 0.024 | 0.011 |
| Catalyst | Pd/C | Pd/C | Pd/C | Pd/C | Pd/C | $PdCl_2$ | Pd/C |
| mg of Catalyst | 50 | 50 | 50 | 50 | 50 | 50 | 50 |
| mg of TPP | 30 | 50 | 30 | 30 | 50 | 50 | 50 |
| Time (hr) | 3 | 4 | 7 | 7 | 12 | 4 | 4 |
| Ratio: PhOH/APhE | 1/98 | 1/99 | 10/90 | 28/72 | 14/86 | 15/85 | 55/7 |
| Comments | | | pH=10.6 | pH=11.6 | He atmosphere | | |

Solvents: IPA = isopropanol
Catalysts: Pd/C = palladium on carbon
Ligands: TPP = triphenylphosphine
Products: PhOH = phenol; APhE = allyl phenyl ether
* - not an example of the invention

Referential Examples 12-18 - Allylation of Bisphenol A

Several different mixtures of bisphenol A and allyl acetate were reacted together under inert atmosphere in 3-neck, round-bottom flasks fitted with a gas inlet, reflux condenser and a thermometer equipped with a temperature controller. The reactor contents were heated to 85°C using an infrared lamp and stirred using a magnetic stirrer. The reagents, the reaction time, the conversion of bisphenol A and the ratio of diallyl ether to monoallyl ether in the product mixture are shown in Table II. Analyses were obtained using high performance liquid chromatography.

TABLE II

| Example | 12 | 13 | 14 | 15 | 16 | 17 | 18 |
|---|---|---|---|---|---|---|---|
| BPA (moles) | 0.75 | 0.75 | 0.025 | 0.025 | 0.01 | 0.01 | 0.01 |
| Allyl Ace. (moles) | 1.8 | 1.8 | 0.06 | 0.06 | 0.06 | 0.06 | 0.06 |
| mL of Water | 0 | 250 | 8 | 8 | 5 | 8 | 5 |
| Base | $K_2CO_3$ | $K_2CO_3$ | $K_2CO_3$ | $K_2CO_3$ | $Na_2CO_3$ | $NaHCO_3$ | $Na_2CO_3$ |
| Moles of Base | 1.8 | 1.8 | 0.058 | 0.058 | 0.03 | 0.03 | 0.015 |
| Catalyst | Pd/C | Pd/C | $Pd/Al_2O_3$ | $Pd/BaSO_4$ | $Pd(Ac)_2$ | $Pd(Ac)_2$ | $Pd(Ac)_2$ |
| mg of Catalyst | 1500 | 1500 | 25 | 25 | 15 | 15 | 15 |
| mg of TPP | 600 | 600 | 25 | 25 | 30 | 30 | 30 |
| Time (hr) | 18 | 9 | 8 | 8 | 2 | 2 | 4 |
| BPA Conversion | 100 | 100 | 98 | 96 | - | - | - |
| Ratio MAE/DAE | 0/100 | 1/99 | 19/79 | 26/70 | 8/90 | 0/100 | 0/100 |

Catalysts: Pd/C = palladium on carbon; Pd/Al2O3 = palladium on alumina; $Pd/BaSO_4$ = palladium on barium sulfate; $Pd(Ac)_2$ = palladium diacetate

Ligands: TPP = triphenylphosphine

Compounds: BPA = bisphenol A; MAE = monoallyl ether of bisphenol A; DAE = diallyl ether of bisphenol A

Referential Examples 19-21

Mixtures containing 60 g (0.6 mole) of allyl acetate, 45 g (0.2 mole) of bisphenol A, 150 mL of organic solvent and 100 mL of water were placed in a glass reactor equipped with glass baffles under nitrogen atmosphere and heated to 80°C. A mixture containing 28 mg of palladium diacetate and 162 mg of triphenylphosphine was added to the reaction mixture. After stirring at 400 rpm for a short time, the temperature was raised to 85°C and a 50 percent aqueous solution of sodium hydroxide was added continuously to the reaction mixture for the time shown in Table III. After the addition was completed, the reaction mixture was analyzed by gas chromatography. The results are shown in Table III:

TABLE III

| Example | 19 | 20 | 21 |
|---|---|---|---|
| Solvents | t-AA | DMEE | t-AA |
| g(mole) of 50% NaOH | 48 (0.6) | 48 (0.6) | 38.4 (0.48) |
| Time (hr) | 2.5 | 2.5 | 2.7 |
| Product Composition (%) | BPA - 0.2<br>MAE - 0.26<br>DAE - 98.7<br>Other - 0.8 | BPA - 0.23<br>MAE - 0.35<br>DAE - 98.8<br>Other - 0.6 | BPA - 0.2<br>MAE - 0.15<br>DAE - 98.9<br>Other - 0.7 |

BPA = bisphenol A; MAE = monoallyl ether of bisphenol A; DAE = diallyl ether of bisphenol A; t-AA = t-amyl alcohol; DMEE = dimethoxyethyl ether

## Claims

**1.** A process for the preparation of an epoxy-containing compound comprising the steps of:

(1) providing an allyl reagent;

(2) allylating one or more compounds containing at least one active-hydrogen atom per molecule using the allyl reagent from Step (1), whereby an allyl ether, allyl thioether or allyl aryl amine compound is formed; and

(3) converting the allyl groups on the compound from Step (2) to epoxide groups, whereby a glycidyl ether, glycidyl thioether or glycidyl aryl amine compound is formed,

characterized in that the allyl reagent is an allyl carboxylate and in that Step (2) is catalyzed by a transition metal catalyst.

**2.** A process as claimed in Claim 1, wherein the allyl carboxylate is made in Step (1) by reacting propylene, oxygen and a carboxylic acid in the presence of a catalyst.

**3.** A process as claimed in any one of the preceding Claims wherein the active-hydrogen-containing compound contains one or more hydroxyl groups, and the product is a glycidyl ether compound.

**4.** A process as claimed in any one of the preceding Claims wherein the active-hydrogen-containing compound contains on average more than 1 but no more than 10 phenolic hydroxyl groups.

**5.** A process as claimed in any one of the preceding Claims, wherein the catalyst in Step (2) contains molybdenum, nickel, palladium or platinum.

**6.** A process as claimed in any one of the preceding claims wherein the reaction mixture in Step (2) further contains a complexing agent for the transition metal catalyst.

7. A process as claimed in Claim 1 wherein the reaction mixture in Step (2) further contains a base in an equivalent ratio of at least 1:1 with respect to the active-hydrogen-containing compound.

8. A process as claimed in Claim 7 wherein the base is an alkali and alkaline earth metal bicarbonate, carbonate, phosphate, citrate, hydroxide or acetate.

9. A process as claimed in Claim 3 wherein Step (3) is carried out using a peroxygen compound.

## Patentansprüche

1. Verfahren zur Herstellung einer epoxyhaltigen Verbindung, umfassend die Stufen von:

   (1) Bereitstellung eines Allylreagens;
   (2) Allylieren einer oder mehrerer Verbindungen, welche wenigstens ein aktives Wasserstoffatom pro Molekül enthalten, unter Verwendung des Allylreagens aus Stufe (1), wodurch eine Allylether-, Allylthioether- oder Allylarylaminverbindung gebildet wird; und
   (3) Umwandlung der Allylgruppen auf der Verbindung von Stufe (2) zu Epoxidgruppen, wodurch eine Glycidylether-, Glycidylthioether- oder Glycidylarylaminverbindung gebildet wird;

   dadurch gekennzeichnet, daß das Allylreagens ein Allylcarboxylat ist, und daß Stufe (2) durch einen Übergangsmetallkatalysator katalysiert wird.

2. Verfahren nach Anspruch 1, bei welchem das Allylcarboxylat in Stufe (1) durch Umsetzung von Propylen, Sauerstoff und einer Carbonsäure in Anwesenheit eines Katalysators hergestellt wird.

3. Verfahren nach einem der vorhergehenden Ansprüche, bei welchem die aktiven Wasserstoff enthaltende Verbindung eine oder mehrere Hydroxylgruppen enthält und das Produkt eine Glycidyletherverbindung ist.

4. Verfahren nach einem der vorhergehenden Ansprüche, bei welchem die aktiven Wasserstoff enthaltende Verbindung im Durchschnitt mehr als 1 aber nicht mehr als 10 phenolische Hydroxylgruppen enthält.

5. Verfahren nach einem der vorhergehenden Ansprüche, bei welchem der Katalysator in Stufe (2) Molybdän, Nickel, Palladium oder Platin enthält.

6. Verfahren nach einem der vorhergehenden Ansprüche, bei welchem das Reaktionsgemisch in Stufe (2) weiter einen Komplexbildner für den Übergangsmetallkatalysator enthält.

7. Verfahren nach Anspruch 1, bei welchem das Reaktionsgemisch in Stufe (2) weiter eine Base in einem Äquivalentverhältnis von wenigstens 1:1, bezogen auf die aktiven Wasserstoff enthaltende Verbindung, enthält.

8. Verfahren nach Anspruch 7, bei welchem die Base ein Alkali- und Erdalkalimetallbikarbonat, -carbonat, -phosphat, -citrat, -hydroxid oder -acetat ist.

9. Verfahren nach Anspruch 3, bei welchem Stufe (3) unter Verwendung einer Peroxidverbindung durchgeführt wird.

## Revendications

1. Procédé de préparation d'un composé époxy, qui comprend les étapes consistant à :

   (1) fournir un réactif d'allylation,
   (2) allyler, au moyen du réactif d'allylation de l'étape (1), un ou plusieurs composés contenant au moins un atome d'hydrogène actif par molécule, en formant ainsi un éther allylique, un thio-éther allylique ou une allylarylamine, et
   (3) transformer les groupes allyle du composé provenant de l'étape (2) en groupe époxy, en formant ainsi un éther glycidylique, un thio-éther glycidylique ou une glycidylarylamine,

procédé caractérisé en ce que le réactif d'allylation est un carboxylate d'allyle et en ce que l'étape (2) est catalysée par un catalyseur à base de métal de transition.

**2.** Procédé selon la revendication 1, dans lequel le carboxylate d'allyle est préparé dans l'étape (1) par réaction du propylène, de l'oxygène et d'un acide carboxylique, en présence d'un catalyseur.

**3.** Procédé selon la revendication 1 ou 2, dans lequel le 'composé à hydrogène actif contient un ou plusieurs groupes hydroxyle et le produit obtenu est un éther glycidylique.

**4.** Procédé selon l'une quelconque des revendications précédentes, dans lequel le composé à hydrogène actif contient en moyenne plus d'un et pas plus de 10 groupes hydroxyle phénolique.

**5.** Procédé selon l'une quelconque des revendications précédentes, dans lequel le catalyseur pour l'étape (2) contient du molybdène, du nickel, du palladium ou du platine.

**6.** Procédé selon l'une quelconque des revendications précédentes, dans lequel le mélange réactionnel de l'étape (2) contient en outre un agent complexant pour le catalyseur à base de métal de transition.

**7.** Procédé selon la revendication 1, dans lequel le mélange réactionnel de l'étape (2) contient en outre une base, en une quantité correspondant à un rapport des équivalents par rapport au composé à hydrogène actif d'au moins 1:1.

**8.** Procédé selon la revendication 7, dans lequel la base est un bicarbonate, un carbonate, un phosphate, un citrate, un hydroxyde ou un acétate de métal alcalin ou de métal alcalino-terreux.

**9.** Procédé selon la revendication 3, dans lequel l'étape (3) est réalisée au moyen d'un composé peroxygéné.